# EUROPÄISCHE PATENTANMELDUNG

(11) **EP 2 486 848 A1**
(43) Veröffentlichungstag der Anmeldung: **15.08.2012**
(21) Anmeldenummer: 12154794.7
(22) Anmeldetag: 09.02.2012
(51) Int. Cl.: A61B 5/024, A61B 5/0456

(54) **Vorrichtung und Verfahren zur Messung der Erholung und des Leistungspotentials einer Testperson**

(30) Priorität: 09.02.2011 AT 1692011
(71) Anmelder: Klepp, Nikolaus, 4810 Gmunden (AT)
(72) Erfinder: Klepp, Nikolaus, 4810 Gmunden (AT)
(74) Vertreter: Jell, Friedrich

(57) **Zusammenfassung**

Die Erfindung beschreibt ein Verfahren und eine Vorrichtung zur Messung der Erholung und des Leistungspotentials einer Testperson, wobei die Herzratenvariabilität (HRV) gemessen wird. In zumindest einer ersten Phase in einer Messung in einer ersten Position werden die Herzratenvariabilität und gegebenenfalls die Pulsfrequenz gemessen und in zumindest einer zweiten Phase in einer Messung in einer zweiten von der ersten unterschiedlichen Position insbesondere unter Vorgabe einer Atemfrequenz werden ebenfalls die Herzratenvariabilität und gegebenenfalls die Pulsfrequenz gemessen, wobei durch eine Analyse der Messwerte der Herzratenvariabilitäten der beiden Phasen der Erholungszustand und das Leistungspotential der Testperson ermittelt werden.

## Beschreibung

Die Erfindung betrifft ein Verfahren zur Messung der Erholung und des Leistungspotentials einer Person, wobei die Herzratenvariabilität (HRV) gemessen wird sowie eine Vorrichtung zur Durchführung des erfindungsgemäßen Verfahrens.

Für die Erstellung effizienter Aktivitätspläne bzw. für die Festlegung des Zeitpunkts des optimalen Leistungsniveaus einer Person stehen mehrere Methoden zur Verfügung. Unter anderem ist für sportmedizinische Anwendungen die Frage eines optimalen Trainingsausmaßes bzw. Trainingszeitpunkts einer trainierenden Person von zentraler Bedeutung. Aber auch in der Rehabilitation von Patienten nach physischen oder psychischen Erkrankungen bzw. in der Arbeitsmedizin ist es wichtig den Erholungszustand des Körpers zu kennen um neue Reize zu setzen oder zu pausieren. Auch die Auswirkungen von Allergenen können gemessen und evaluiert werden.

Nur eine Pulsfrequenzmessung ist hierzu jedoch ungeeignet, da die Pulsfrequenz annähernd proportional zur Belastungsintensität ist und sich daraus ein optimales Belastungsniveau nicht bestimmen lässt.

Auch Ruhepulsmessungen werden gerne zur Aktivitätskontrolle verwendet. So wird aufgrund eines niedrigen Ruhepulses auf eine gute Erholung und aufgrund eines hohen Ruhepulses auf eine schlechte Erholung geschlossen. Diese Methode ist zwar sehr einfach in der Anwendung funktioniert aber nur morgens unmittelbar nach dem Aufwachen. Zudem erfolgt die Messung meist zu kurz und Schwankungen werden oft überbewertet.

Ferner ist aus dem Stand der Technik bekannt Methoden anzuwenden, wo mittels Pulsfrequenz und statistischen Daten auf die Leistungsfähigkeit geschlossen wird, wie beispielsweise durch Polar OwnZoneTM. Auch hier erfolgt die Messung meist über einen kurzen Zeitraum, die Kalibrierung erfolgt statistisch und es können methodisch bedingte Scheinkorrelationen entstehen, wobei das Ergebnis für eine individuelle Person nicht stimmig ist.

Es ist bekannt, dass die Variabilität des Herzrhythmus ein Gradmesser der Gesundheit ist. Die Variabilität ist ein Maß für die Änderung der zeitlichen Abstände zwischen zwei Herzschlägen. Im Allgemeinen sprechen größere Schwankungen für eine höhere vegetative Regulationsfähigkeit des Organismus und damit für eine stärkere Lebensenergie. Schwankungen des Herzrhythmus ermöglichen die Analyse des vegetativen Nervensystems. Der Funktionszustand des autonomen Nervensystems lässt sich am effizientesten durch eine Herzanalyse feststellen. Die Veränderung des Herzrhythmus ist eine universelle Reaktion des gesamten Organismus auf beliebige Einwirkungen der Umwelt und Umgebung. Die traditionell gemessene mittlere Herzfrequenz spiegelt jedoch nur den "Endeffekt" der zahlreichen Regulationseinwirkungen auf das Herz-Kreislauf-System wider. Zwei Menschen mit gleicher durchschnittlicher Herzfrequenz können folglich in einem unterschiedlichen vegetativen Regulationszustand sein. Das heißt die gleiche durchschnittliche Herzfrequenz kann unterschiedliche Aktivitätskombinationen der Kettenglieder des die vegetative Homöostase steuernden Systems entsprechen. Messbar wird dies über die Herzratenvariabilität (HRV). Die Variabilität des Herzrhythmus bezieht sich auf den Grad der Schwankung in der Länge der Intervalle zwischen den Herzschlägen. Die bekannteste Herzrhythmusvariabilität ist die respiratorische Sinusarrhythmie. Hierbei kommt es zur Verkürzung der Dauer der RR-Intervalle bei der Einatmung und zur Verlängerung der Dauer der RR-Intervalle bei der Ausatmung. Die Analyse der Herzratenvariabilität wird in klassischer Weise in drei Grafiken dargestellt: dem Tachogramm, dem Histogramm (ergibt sich aus dem EKG und dem Intervall-Tachogramm, einer Serie von Blutdruckintervallen) und dem Streudiagramm. Nach Herzinfarkt, Schlaganfall und bei chronischen Erkrankungen ist die vegetative Regulation eingeschränkt oder blockiert, von Burn-out über Depression bis hin zum metabolischen Syndrom können die Ursachen in einer Dysbalance vegetativer Funktionen nachgewiesen werden. Der wichtigste Parameter bei solchen Funktionsstörungen ist die Variabilität der Herzfrequenz als Ausdruck der Regulationsfähigkeit. Die Herzfrequenzvariabilität ist der wichtigste Parameter zur exakten Beurteilung der funktionalen Vitalität von Gesundheit und Wohlbefinden (aus "Die Variabilität des Herzrhythmus, Raum & Zeit, Sonderdruck, Prof. Dr. Kai Börnert und Dr. Michael Süß").

Aufgabe der vorliegenden Erfindung ist es die Erholung und das Leistungspotential einer Testperson zu analysieren.

Die Aufgabe der vorliegenden Erfindung wird jeweils eigenständig durch ein Verfahren gelöst, wobei die Herzratenvariabilität (HRV) und gegebenenfalls die Pulsfrequenz in einer ersten Phase in einer ersten Position und in einer zweiten Phase in einer zweiten von der ersten unterschiedlichen Position unter Vorgabe der Atemfrequenz gemessen werden, wobei durch eine Analyse der Messwerte der Herzratenvariabilitäten der beiden Phasen der Erholungszustand und das Leistungspotential ermittelt werden, sowie durch eine Vorrichtung mit einem am Körper befestigbaren Sensor zur Aufzeichnung der RR-Intervalle zur Messung der Herzratenvariabilität zur Durchführung des oben genannten Verfahrens und einer Auswerteeinheit zur Analyse der aus dem Verfahren generierten Daten gelöst. Vorteilhaft dabei erweist sich, dass die Erholung, insbesondere der Erholungszustand, das Ausmaß der Erholung bzw. die Regenerationsfähigkeit gemessen werden können und somit das Aktivitätsausmaß auf die Regenerationsfähigkeit und das Leistungspotential einer Person abgestimmt werden kann. Die Herzratenvariabilität ist eine optimale Methode zur Beurteilung der Regenerationsfähigkeit. Sie ist äußerst einfach hinsichtlich des Informationsgewinnes, weil sie lediglich eine hinreichend genaue Messung der RR-Intervalle erfordert. Daraus werden die aufeinander folgenden RR-Intervalle analysiert. Durch die Datengewinnung der Herzratenvariabilität ermöglicht das erfindungsgemäße Verfahren umfangreiche Auskunft über die Regenerationsfähigkeit des Organismus. Vorteilhaft dabei erweist sich auch die Kopplung der Herzratenvariabilität mit der Atemfrequenz in der zweiten Phase, weil somit auch der Einfluss anderer Größen, wie des Gesamtzustands und der Umwelt berücksichtigt werden können. Durch die Vorgabe der Atemfrequenz kann auch eine Belastung simuliert werden.

Es konnte sich für das erfindungsgemäße Verfahren herausstellen, dass eine Atemvorgabe, insbesondere eine von einer weiteren Messung wesentlich abweichende Atemvorgabe von Vorteil sein kann. Vielfach konnte jedoch festgestellt werden, dass diese nicht zwingend erforderlich ist, es jedoch vorteilhaft sein kann. Zu erwähnen ist in diesem Zusammenhang unter anderem, dass als, von einer anderen Messung wesentlich abweichende Atemvorgabe, in manchen Fällen eine Änderung der Position der Testperson ausreichte, beispielsweise ein Wechsel von einer sitzenden in eine liegende, um das erfindungsgemäße Verfahren durchzuführen. Hingegen konnte bei anderen Personen, insbesondere bei solchen, die sich in einem vergleichsweise guten körperlichen Zustand befanden, eine stärker abweichende Atemvorgabe die Durchführung des erfindungsgemäßen Verfahrens erleichtern und/oder verbessern.

Wird die Herzratenvariabilität während eines vordefinierten Zeitraums oder einer vordefinierten Anzahl von Pulsschlägen gemessen ist sichergestellt, dass die aus diesen Messungen gewonnen Daten mit Daten aus nachfolgenden Messungen unter den gleichen Bedingungen vergleichbar sind.

In der ersten Position wird die Herzratenvariabilität im Liegen gemessen, wodurch während eines entspannten und mehr oder weniger von Umwelteinflüssen ungestörten Rahmens die Herzratenvariabilität bestimmt wird.

Vorzugsweise erfolgt die Messung der Herzratenvariabilität in der ersten Position für eine Zeitdauer von 3 bis 10 Minuten, in der zweiten Position für eine Zeitdauer von 3 bis 5 Minuten und gegebenenfalls in zumindest einer dritten Position für einen Zeitraum von 1 bis 3 Minuten oder länger, wodurch eine repräsentative Anzahl von RR-Intervallen für die Auswertung zur Verfügung steht.

Der Vollständigkeit halber wird erwähnt, dass für die erfindungsgemäße Analyse - wie obenstehend beschrieben - vorzugsweise RR-Intervalle herangezogen werden können, hierfür aber selbstverständlich auch andere Parameter vorstellbar sind. Erwähnt seien in diesem Zusammenhang beispielsweise die Abstände zwischen Pulswellen, deren Bestimmung etwa durch entsprechende Messung an Ohr oder Fingerkuppe möglich ist.

Die zweite Messung der Herzratenvariabilität erfolgt vorzugsweise unter einer definierten Belastung, wie beispielsweise im Sitzen oder Stehen, womit auch der Einfluss einer anderen Position, insbesondere einer Belastung, in der Auswertung der Herzratenvariabilität und in weiterer Folge auf das Potential der Testperson berücksichtigt werden kann. In Kombination mit dem Ergebnis der ersten Messung kann sowohl auf den Erholungszustand als auch auf das Leistungspotential der Testperson geschlossen werden. Die Messung in der zweiten Position erfolgt vorzugsweise nach der ersten Messung, vorzugsweise in mehr oder weniger unmittelbar aufeinander folgenden Phasen. Zuerst erfolgt in der ersten Phase die Ruhemessung im Liegen, danach ist eine Pause zum Wechsel der Position zum Aufsitzen vorgesehen, und anschließend erfolgt in der zweiten Phase die Belastungsmessung im Sitzen. Um die Wirkung einer Tätigkeit, z.B. eines Trainings, zu bestimmen, kann man vor und nach der Belastung messen, beispielsweise 30 Minuten vorher und eine Stunde nachher. Bei Anwendungen des erfindungsgemäßen Verfahrens im arbeitsmedizinischen Bereich kann beispielsweise die Auswirkung einer bestimmten Tätigkeit oder eines Schichtbetriebs auf die Testperson gemessen werden. Im Rehabilitationsbereich kann durch das erfindungsgemäße Verfahren das Ansprechen eines Patienten auf die Therapie überprüft werden. Mit diesen zwei bzw. drei Messwertpaaren kann man eine Aussage über den Einfluss einer Aktion, wie körperliche Betätigung, psychische Belastung, aber auch von Nahrungsmitteln, Allergenen, Toxinen, Stress, etc. auf die Testperson treffen.

Der Wert der Atemfrequenzvorgabe wird aus einem Bereich mit einer unteren Grenze von 4 und einer oberen Grenze von 30, vorzugsweise 8 bis 12, Herzschläge/Atemzug ausgewählt, wodurch der Durchschnittspuls reguliert werden kann und in weiterer Folge eine induzierte Änderung der Herzratenvariabilität erfolgt. Aus der Form der Antwortfunktion der Pulskurve auf die Atemvorgabe kann auf einen zu hohen oder zu kleinen Kopplungsfaktor geschlossen werden.

Vorzugsweise wird bei unterschiedlichen Messungen der Wert der Atemvorgabe konstant gehalten, womit vergleichbare Parameter bei wiederholten Messungen für die Auswertung vorliegen. Dieser Wert kann ein einziges Mal individuell bestimmt werden und ist in allen nachfolgenden Messungen gleich. Im Prinzip wird die Antwortfunktion auf die Atemvorgabe (z.B. Sinusfunktion) untersucht.

Es kann auch zumindest eine dritte Messung der Herzratenvariabilität in einer dritten Position, welche sich von der ersten und zweiten Position unterscheidet und vorzugsweise eine Aktivitätsposition umfasst, erfolgen, womit auch der unmittelbare Einfluss während der Aktivität bzw. der Anstrengung auf die Herzratenvariabilität gemessen werden kann bzw. in der Festlegung des Aktivitätsplans berücksichtigt werden kann.

Zum besseren Verständnis der Erfindung wird diese anhand der nachfolgenden Figuren näher erläutert.

Es zeigen jeweils in stark vereinfachter schematischer Darstellung:
- Fig. 1: graphische Darstellung der Auswertung der Herzratenvariabilität eines Sportlers;
- Fig. 2: graphische Darstellung der Auswertung der Herzratenvariabilität eines Schichtarbeiters;
- Fig. 3: Darstellung der Herzratenvariabilität einer Testperson mittels Scatterplot.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiter können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1 oder 5,5 bis 10. Ferner wird darauf hingewiesen, dass alle Werte beispielhaft zu verstehen sind.

Im Zusammenhang mit der Erfindung wird unter Messung der Herzratenvariabilität die Messung der RR-Intervalle und daraus die Berechnung der Herzratenvariabilität verstanden.

Mit dem erfindungsgemäßen Verfahren lässt sich durch die Messung der Herzratenvariabilität die Erholung bzw. Regenerationsfähigkeit und das Potential, insbesondere Leistungspotential, eines Organismus bestimmen. So kann der Einfluss diverser Tätigkeiten, Aktivitäten, Therapien, Nahrungsmittel, Allergene, etc. auf den Organismus gemessen werden. Die Herzratenvariabilität beschreibt die Schwankungen der RR-Intervalle.

Mit dem erfindungsgemäßen Verfahren kann beispielsweise die Regenerations- bzw. Leistungsfähigkeit als Mittel zur Trainingsoptimierung herangezogen werden, wobei ein neuartiger Lösungsweg verfolgt wird. Es ist bekannt, dass Atmung und Pulsfrequenz gekoppelt sind, wobei, sofern eine Größe geändert wird, sich auch die andere verändert. Die beiden Größen Atmung und Pulsfrequenz werden durch den physiologischen Gesamtzustand und die Umwelt definiert bzw. beeinflusst. Mit dem erfindungsgemäßen Verfahren ist es nunmehr möglich, den physiologischen Gesamtzustand zu messen. Ferner kann auch der Einfluss einer Psycho- oder Physiotherapie, einer rehabilitativen Maßnahme, eines Nahrungsmittels, Stress, Allergens, etc. auf den Organismus gemessen werden und somit die weitere therapeutische Vorgehensweise abgestimmt werden.

Es werden RR-Intervalle in zwei oder mehr Phasen gemessen und vorzugsweise Wertepaare analysiert.

Hierbei wird in einer ersten Phase in einer ersten Messung in einer ersten Position der erste Wert zur Berechnung der Herzratenvariabilität und gegebenenfalls der Pulsfrequenz ermittelt. Diese erste Messung erfolgt vorzugsweise in einem vordefinierten Zeitraum bzw. während einer vordefinierten Anzahl von Pulsschlägen. Insbesondere erfolgt die Messung für eine Zeitdauer von 5 bis 10 min. Als bevorzugte Position während der ersten Messung wird die Position des Liegens gewählt, weil hierbei die Entspannung am größten ist. So kann beispielsweise morgens unmittelbar nach dem Aufwachen die erste Messung durchgeführt werden. Es ist auch möglich nach Konsumation bestimmter Nahrungsmittel, nach erfolgtem Training bzw. nach erfolgter Tätigkeit, wie beispielsweise Arbeit, die erste Messung in entspannter Position im Liegen durchzuführen.

Hierbei spiegelt das Ergebnis der ersten Messung der Herzratenvariabilität in Ruhe den Ist-Zustand, wobei wiederum je höher die Herzratenvariabilität ist, umso besser ist der Ist-Zustand (Gesamtzustand) der Testperson, also umso besser ist die Erholung der Testperson auf eine Aktivität, Nahrungsmittel, Therapie, etc.

In einer zweiten Phase erfolgt die Ermittlung des zweiten Wertes des Wertepaares, also die zweite Messung, die in einer zweiten von der ersten unterschiedlichen Position durchgeführt wird. Während der Ermittlung des zweiten Wertes wird die Atemfrequenz vorgegeben. Diese zweite Messung erfolgt vorzugsweise im Sitzen bzw. in einem anderen Belastungszustand. Die zweite Messung wird für einen Zeitraum von 3 bis 5 min durchgeführt. Sie kann auch, wie bereits vorab beschrieben, unmittelbar nach einer Belastung bzw. Nahrungsaufnahme bzw. einer anderen Tätigkeit erfolgen. Diese zweite Messung der Herzratenvariabilität kann beispielsweise auch nach einer emotionalen Belastung durchgeführt werden.

Die Zeitdauer sowohl für die erste als auch die zweite Messung kann gegebenenfalls auch kürzer, z.B. 30 Sekunden, oder länger sein, z.B. 60 Minuten bis zu 24 oder 48 Stunden. Erfolgt die Messung über einen langen Zeitraum kann auch die Position der Testperson geändert werden bzw. variieren.

Die Messung der ersten und zweiten Phase wird vorzugsweise morgens und abends durchgeführt, wobei die daraus resultierenden Werte über einen längeren Zeitraum beobachtet werden, um entsprechende Auswertungen und Aussagen treffen zu können.

Die erste Messung kann beispielsweise morgens noch im Liegen erfolgen und ca. 15 Minuten nach Änderung der Position ins Sitzen kann die zweite Messung durchgeführt werden. In einer bevorzugten Ausführungsvariante liegt die Testperson entspannt und es kann ein Sensortest der am Körper befestigbaren Einheit mit einem Sensor zur Aufzeichnung der Herzratenvariabilität bzw. Herzfrequenz bzw. des Brustgurts erfolgen. Anschließend findet gegebenenfalls eine Pause statt und es folgt die Entspannungsphase, vorzugsweise im Liegen, zur Messung der RR-Intervalle und somit Berechnung der Herzratenvariabilität der ersten Phase. Nach der ersten Messung in der ersten Position ändert die Testperson die Position und wechselt in eine sitzende Position, in welcher in der Belastungsphase unter einer Atemfrequenzvorgabe die Messung der RR-Intervalle und somit Berechnung der Herzratenvariabilität der zweiten Phase stattfindet.

Eine alternative Anwendung des erfindungsgemäßen Verfahrens ist die erste Messung am Morgen im Liegen durchzuführen und die zweite Messung irgendwann nach einer Belastung bzw. nach einem Positionswechsel durchzuführen.

Eine weitere Variante ist die erste Messung am Morgen im Liegen durchzuführen und die zweite Messung ebenfalls am Morgen aber des nächsten Tages im Liegen durchzuführen. Die zweite Messung erfolgt unter einer Atemfrequenzvorgabe.

Der Wert der Atemfrequenzvorgabe ist aus einem Bereich mit einer unteren Grenze von 4 und einer oberen Grenze von 30 Herzschlägen pro Atemzug, der aus einer Aus- und Einatmung besteht, ausgewählt. Meist liegt die Atemfrequenzvorgabe zwischen 8 und 12 Herzschläge pro Atemzug. Bei dem erfindungsgemäßen Verfahren dient die Atemvorgabe dazu die Durchschnittspulsfrequenz künstlich niedrig zu halten, nämlich auf etwa demselben Wert wie in der ersten Phase. Die Messung der Herzratenvariabilität ist somit ein Maß für die Beeinflussbarkeit des Herz-KreislaufSystems. Im Prinzip gilt, dass je höher die Belastung oder der Stress ist umso geringer ist die Herzratenvariabilität. Dies ist ein Maß für die Regenerationsfähigkeit bzw. das Leistungspotential.

Vorzugsweise wird während der Dauer der zweiten Messung der Wert der Atemfrequenzvorgabe konstant gehalten. Die Periodendauer in Abhängigkeit von der durchschnittlichen RR-Intervalllänge wird empirisch vor Durchführung der Messreihen ermittelt. Sie wird aus dem Durchschnittspuls der vorhergehenden Phase berechnet. Auch hier gilt je höher die Herzratenvariabilität ist, umso besser ist der Gesamtzustand der Person. Gleichzeitig ist die Herzratenvariabilität auch ein Maß für die zu erwartende Erholung der Person. Durch diese zweite Messung wird auch die Fähigkeit zur Einflussnahme auf die Kontrollsysteme bestimmt.

Um das Allgemeinbefinden einer Testperson zu überwachen kann die erste Messung auch als Morgenmessung und die zweite Messung als Abendmessung durchgeführt werden, wobei beide Messungen ohne Atemfrequenzvorgabe durchgeführt werden können bzw. die zweite Messung unter Vorgabe der Atemfrequenz erfolgt.

Es können auch noch weitere Messungen durchgeführt werden, die beispielsweise in einer dritten Position erfolgen, welche sich von der ersten und zweiten Position unterscheidet und vorzugsweise während einer körperlichen Beanspruchung durchgeführt wird. Die Zeitdauer für die dritte Messung der Herzratenvariabilität liegt zwischen 1 und 3 min bzw. kann während der gesamten Dauer der dritten Position, insbesondere während des Trainings, durchgeführt werden.

Es ist empfehlenswert nach einem Positionswechsel zumindest 30 sec, vorzugsweise 1 min, abzuwarten, bevor die Messung in der neuen Position durchgeführt wird.

Die Messung in der ersten Position wiederspiegelt den Erholungszustand am Morgen bzw. nach einer Erholungsphase. In Korrelation mit der Aktivität, Nahrungsmittel, Therapie, Trainingsleistung, etc. ergibt sich daraus die Regenerationsfähigkeit des Organismus.

Ferner können mit dem erfindungsgemäßen Verfahren auch Auswirkungen von Nahrungsmitteln (Wein, Kaffee, Essen) direkt gemessen werden. Es kann auch festgestellt werden, ob eine Tätigkeit erholsam ist oder nicht, bzw. wie sich Trainingseinheiten auswirken. Ein weiterer Effekt der Messungen ist, dass die Messung entspannend ist, weil die Person zur Ruhe kommt.

Weiter umfasst die Erfindung eine Vorrichtung zur Messung des Leistungspotentials einer Person umfassend eine am Körper befestigbare Einheit mit einem Sensor zur Aufzeichnung der Herzratenvariabilität, wobei eine Auswerteeinheit zur Analyse der aus dem erfindungsgemäßen Verfahren generierten Daten umfasst ist. Beispielsweise kann die Vorrichtung eine am Körper befestigbare Einheit mit einem Sensor zur Aufzeichnung der Herzratenvariabilität bzw. Herzfrequenz bzw. einen Brustgurt umfassen, der mit einem Sensor zur Aufzeichnung der RR-Intervalle zur Messung der Herzratenvariabilität ausgestattet ist, und eine Überwachungseinheit, die ähnlich einer Armbanduhr von einer Person getragen werden kann. Der Sensor weist Elektroden auf, sodass in an sich bekannter Weise ein Elektrokardiogramm der Herzaktivität der Person aufgezeichnet werden kann. Es kann allerdings auch eine EKG genaue Pulsmessung durchgeführt werden und in weiterer Folge die Herzratenvariabilität bestimmt werden.

Die Bestimmung der Herzratenvariabilität erfolgt unter anderem wie im Patent der Anmelderin EP 2 120 691 B1 beschrieben.

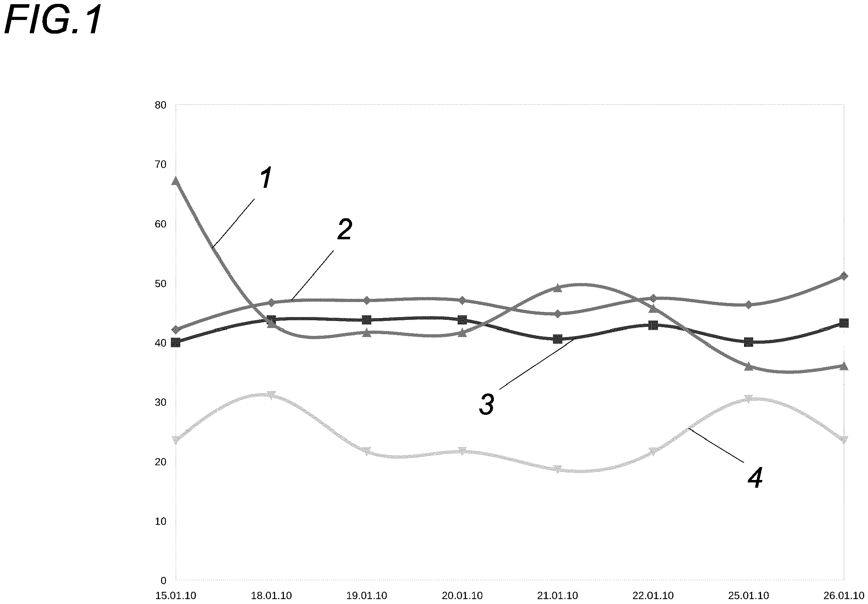

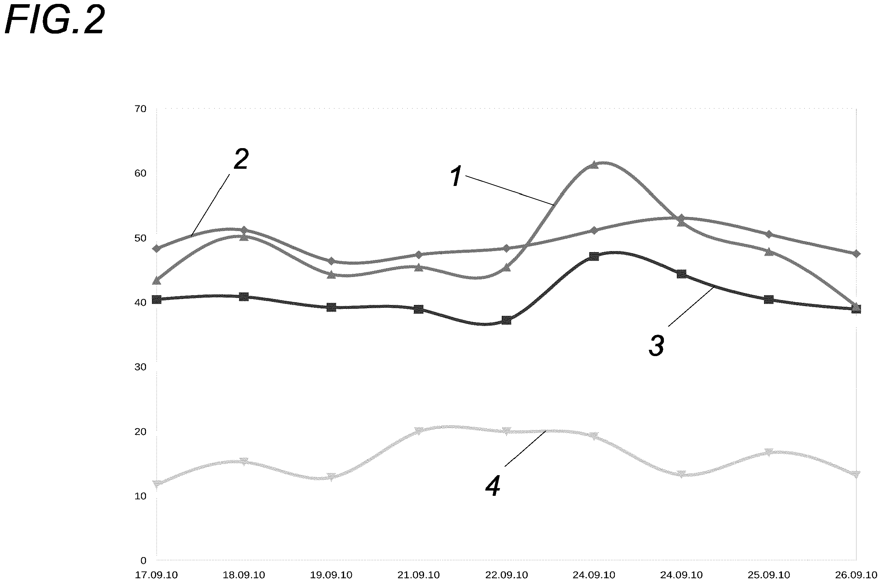

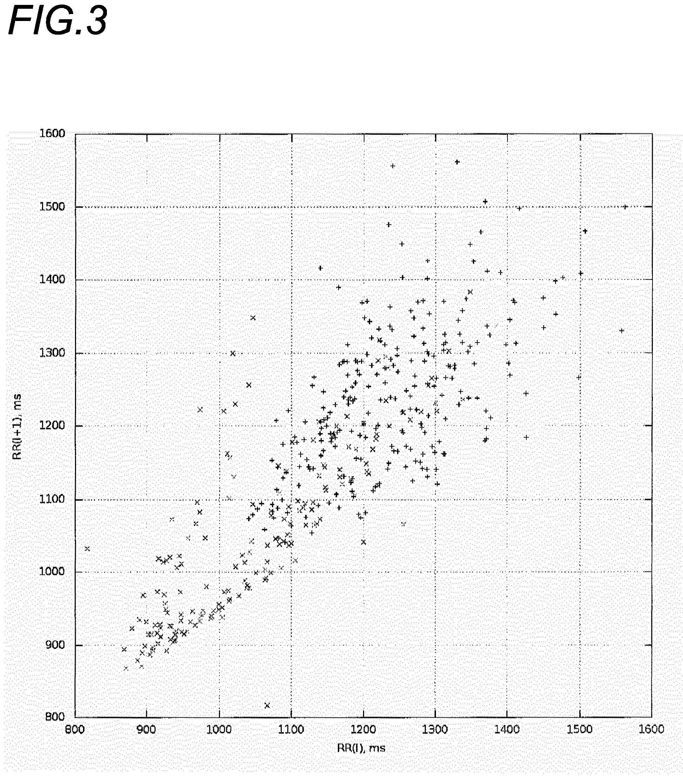

Über die Messungen der RR-Intervalle der ersten und zweiten, gegebenenfalls auch dritten, Phase wird ein Bandfilter gelegt. Aus den Werten des ausgewählten Bereichs wird ein Scatterplot, wie beispielsweise in Fig. 3 gezeigt, erstellt. Dabei werden Wertepaare aus aufeinander folgenden RR-Intervallen als Punkte in den Plot eingetragen. In dieser zweidimensionalen grafischen Darstellung ergibt sich somit eine Punktwolke, in der die Variabilität der Herzfrequenz in einer Weise zum Ausdruck kommt, die eine weitergehende Analyse erlaubt. Es werden zu den Punkten der Punktwolke eine Hauptlängenachse X0 und eine Hauptbreitenachse Yo berechnet. X ist im Diagramm die horizontale Achse (1,0) = 0? Y ist im Diagramm die vertikale Achse ( 0,1) = 90? X0 entspricht dem Vektor (x,y)=(1,1) also 45°.Y0 entspricht dem Vektor (x,y)=(-1,1) also 135? Dies kann mittels Methoden der Regressionsanalyse bzw. orthogonaler Regressionsanalyse erfolgen. Es wird aus den orthogonalen Abständen eine Standardabweichung SOL bzw. SOW berechnet. SOL und SOw sind die Standardabweichungen der Punkte in Richtung X0 und Yo, also nach der Koordinatentransformation. Das Produkt aus SOL mit SOw normiert auf den Mittelwert der RR-Intervalle (SOL"SOw/RR_MED2) aus der ersten Phase ergibt den Wert A und das Produkt aus SOL und SOw normiert auf den Mittelwert der RR-Intervalle aus der zweiten Phase ergibt den Wert B. Diese Werte können über einen Beobachtungszeitraum in ein Diagramm aufgetragen werden und analysiert werden. Solche Diagramme sind in Fig. 1 und 2 dargestellt.

Der Wert der Herzratenvariabilität sinkt mit zunehmendem Alter. Je niedriger die Herzratenvariabilität ist, umso geringer sind die Erholung und das Leistungspotential einer Person.

Durch die Messung der Herzratenvariabilität mit dem erfindungsgemäßen Verfahren ist der Erholungszustand messbar, wobei die Werte konsistent reproduzierbar sind. Es zeigte sich, dass gleiche Belastungen gleiche Auswirkungen bei einer Person haben. Auch konnte gezeigt werden, dass körperliche Belastung, wie beispielsweise Schlafmangel, Spuren, also eine Beeinflussung des Wertes der Herzratenvariabilität, hinterlässt. Ferner erlauben Messungen im Tagesverlauf Beobachtungen der Regenerationsfähigkeit und somit kann in weiterer Folge die Aktivität auf die Regenerations- und Leistungsfähigkeit abgestimmt werden.

Durch den Wert der zweiten Phase kann eine Prognose und somit die Regenerationsfähigkeit vorhergesagt werden. Der Wert der ersten Phase spiegelt hingegen die Erholung, insbesondere den Gesamtzustand, der Person, wobei sowohl der psychische als auch der physische Zustand evaluiert werden. Bei der Auswertung beider Werte ist es wichtig, den Verlauf der beiden Messungen zu kontrollieren.

### Ausführungsbeispiel 1

Fig. 1 zeigt die Auswertung einer Trainingswoche einer Person, wobei die Messungen jeweils morgens durchgeführt wurden. Der jeweilige Tag der Messung ist auf der X-Achse definiert. Die Kurve 4 zeigt Ergebnisse der Analyse der Herzratenvariabilität unter Entspannung, während die Kurve 1 die Herzratenvariabilität unter einer bestimmten Atemvorgabe wiederspiegelt. Die Ergebnisse der Pulsfrequenz während Entspannung werden als Kurve 3, jene der Pulsfrequenz, die sich unter Atemvorgabe einstellte, werden als Kurve 2 dargestellt. In diesem Ausführungsbeispiel wurde Entspannung als liegende Position und Atemvorgabe als sitzende Position definiert. Es konnte gezeigt werden, dass während der gesamten Trainingswoche kein Übertraining erfolgte, aber generell niedrige Absolutwerte bezüglich der Herzratenvariabilität vorliegen. Diese Aussage kann getroffen werden, weil keine großen Schwankungen in der Herzratenvariabilität sowohl während der ersten als auch während der zweiten Messung festgestellt werden konnten. Nur aufgrund der Pulsmessung alleine kann eine Aussage bezüglich des Gesamtzustands der Person nicht gemacht werden.

### Ausführungsbeispiel 2

Im Ausführungsbeispiel 2, dessen Ergebnis in Fig. 2 dargestellt ist, werden die Auswirkungen von Schichtarbeit auf eine Person gezeigt. Dazu wurden Messungen vom 17.09.2010 bis inkl. 19.09.2010 jeweils nach einer Spätschicht, am 21. UND 22.09.2010 nach einer Nachtschicht, am 24.09.2010 vor einer Frühschicht und von 24.09.2010 bis 26.09.2010 jeweils nach einer Frühschicht durchgeführt. Es wurden also bewusst Tage für die Analyse gewählt, die aufgrund wechselnder Arbeitszeiten eine besonders hohe Belastung für die Testperson erwarten lassen sollten - unter anderem in Hinblick auf eine Störung des Biorhythmus. Die Definitionen der Kurven 1, 2, 3 und 4 entsprechen jener der Figur 1, ebenso sind Entspannung und Atemvorgabe wie im Ausführungsbeispiel 1 festgelegt. Hier konnte gezeigt werden, dass Frühschichten (FS) sich bezüglich des Wertes der Herzratenvariabilität negativ auswirken. Wohingegen Spätschichten (SS) - im Übrigen konnte dies auch für Nachmittagsschichten (NS) festgestellt werden - keine Auswirkung auf die Herzratenvariabilität bzw. auf den Gesamtzustand und somit die Regenerationsfähigkeit zeigen. Nach der Freizeit - entspricht dem höchsten Wert der Herzratenvariabilität - nahm während der nachfolgenden Frühschichten die Herzratenvariabilität beständig ab und somit reichten die Schlafperioden zur Erholung nicht aus. Ferner konnte durch die untere Kurve 4 - also der Messung der Herzratenvariabilität im Liegen - gezeigt werden, dass die Testperson bereits erschöpft ist, denn die Freizeit war zwar ausreichend, den Stress abzubauen - obere Kurve 1 der Herzratenvariabilität, Messung unter Belastung - aber nicht ausreichend zum Regenerieren. Anderenfalls wäre nach der Freizeit die untere Kurve der Herzratenvariabilität, welche den Ist-Zustand = Gesamtzustand, insbesondere die Erholung, wiedergibt, deutlich höher als beim Ende der letzten Nachtschicht.

### Ausführungsbeispiel 3

In Fig. 3 wird das Ergebnis der Messung der ersten und der zweiten Phase einer unauffälligen Testperson in Form eines Streudiagramms dargestellt. Die Punkte mit der Kennzeichnung "+" stellen die Werte der RR-Intervalle in Entspannung, also der ersten Phase, die Punkte mit der Kennzeichnung "x" stellen die Werte der RR-Intervalle unter Atemvorgabe, also der zweiten Phase, dar.

Die Ausführungsbeispiele zeigen mögliche Ausführungsvarianten des Verfahrens zur Messung des physischen Leistungspotentials einer Person, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Die den eigenständigen erfinderischen Lösungen zugrundeliegende Aufgabe kann der Beschreibung entnommen werden.

## Patentansprüche

1. Verfahren zur Messung der Erholung und des Leistungspotentials einer Testperson, wobei die Herzratenvariabilität (HRV) gemessen wird, **dadurch gekennzeichnet, dass** in zumindest einer ersten Phase in einer Messung in einer ersten Position die Herzratenvariabilität und gegebenenfalls die Pulsfrequenz gemessen werden und in zumindest einer zweiten Phase in einer Messung in einer zweiten von der ersten unterschiedlichen Position, insbesondere unter Vorgabe einer Atemfrequenz, ebenfalls die Herzratenvariabilität und gegebenenfalls die Pulsfrequenz gemessen werden, wobei durch eine Analyse der Messwerte der Herzratenvariabilitäten der beiden Phasen der Erholungszustand und das Leistungspotential der Testperson ermittelt werden.

2. Verfahren nach Anspruch 1, **dadurch gekennzeichnet, dass** die Messwerte der Herzratenvariabilität während eines vordefinierten Zeitraums oder einer vordefinierten Anzahl von Pulsschlägen ermittelt werden.

3. Verfahren nach Anspruch 1 oder 2, **dadurch gekennzeichnet, dass** in der ersten Position im Liegen die Herzratenvariabilität gemessen wird.

4. Verfahren nach einem der Ansprüche 1 bis 3, **dadurch gekennzeichnet, dass** die Messung der Herzratenvariabilität in der ersten Position für eine Zeitdauer von 3 bis 10 Minuten durchgeführt wird.

5. Verfahren nach einem der Ansprüche 1 bis 4, **dadurch gekennzeichnet, dass** die Messung der Herzratenvariabilität in der zweiten Position unter einer Belastung, insbesondere im Sitzen oder Stehen, durchgeführt wird.

6. Verfahren nach einem der Ansprüche 1 bis 5, **dadurch gekennzeichnet, dass** die Messung der Herzratenvariabilität in der zweiten Position für eine Zeitdauer von 3 bis 5 Minuten durchgeführt wird.

7. Verfahren nach einem der Ansprüche 1 bis 6, **dadurch gekennzeichnet, dass** der Wert der Atemfrequenzvorgabe aus einem Bereich mit einer unteren Grenze von 4, vorzugsweise 8, und einer oberen Grenze von 30, insbesondere 12, Herzschläge/Atemzug ausgewählt wird.

8. Verfahren nach einem der Ansprüche 1 bis 7, **dadurch gekennzeichnet, dass** der Wert der Atemfrequenzvorgabe für eine Testperson konstant gehalten wird.

9. Verfahren nach einem der Ansprüche 1 bis 8, **dadurch gekennzeichnet, dass** die Herzratenvariabilität auch zumindest in einer dritten Position, welche sich von der ersten und zweiten Position unterscheidet und vorzugsweise eine Trainingsposition umfasst, gemessen wird.

10. Verfahren nach Anspruch 9, **dadurch gekennzeichnet, dass** die Messung der Herzratenvariabilität in der dritten Position für eine Zeitdauer von 1 bis 3 Minuten oder in etwa der Dauer der dritten Position durchgeführt wird.

11. Verfahren nach einem der Ansprüche 1 bis 10, **dadurch gekennzeichnet, dass** die Erholung und das Leistungspotential eines Sportlers, Berufstätigen und/oder Patienten ermittelt werden.

12. Vorrichtung zur Messung des Leistungspotentials einer Person umfassend eine am Körper befestigbare Einheit mit einem Sensor zur Aufzeichnung der RR-Intervalle zur Messung der Herzratenvariabilität, **dadurch gekennzeichnet, dass** eine Auswerteeinheit zur Analyse der aus dem Verfahren nach einem der Ansprüche 1 bis 11 generierten Daten umfasst ist.
